Europäisches Patentamt

European Patent Office

Office européen des brevets

(19)

(11) **EP 0 652 805 B1**

(12) **EUROPÄISCHE PATENTSCHRIFT**

(45) Veröffentlichungstag und Bekanntmachung des Hinweises auf die Patenterteilung:
08.10.1997 **Patentblatt 1997/41**

(21) Anmeldenummer: 93915943.0

(22) Anmeldetag: 17.07.1993

(51) Int. Cl.$^6$: **B01J 23/89**, B01D 53/56

(86) Internationale Anmeldenummer:
PCT/EP93/01889

(87) Internationale Veröffentlichungsnummer:
WO 94/02244 (03.02.1994 Gazette 1994/04)

(54) **SILBERHALTIGER ALUMINIUMOXID-TRAGERKATALYSATOR UND VERFAHREN ZUR ZERSETZUNG VON DISTICKSTOFFMONOXID**

ALUMINIUM OXIDE CATALYST CONTAINING SILVER, AND METHOD OF DECOMPOSING NITROUS OXIDE

CATALYSEUR A SUPPORT CONSTITUE D'OXYDE D'ALUMINIUM, CONTENANT DE L'ARGENT, ET PROCEDE DE DECOMPOSITION D'OXYDE AZOTE

(84) Benannte Vertragsstaaten:
BE DE ES FR GB IT NL

(30) Priorität: 28.07.1992 DE 4224881

(43) Veröffentlichungstag der Anmeldung:
17.05.1995 Patentblatt 1995/20

(73) Patentinhaber: BASF Aktiengesellschaft
67063 Ludwigshafen (DE)

(72) Erfinder:
• FETZER, Thomas
D-6700 Ludwigshafen (DE)
• BUECHELE, Wolfgang
D-6700 Ludwigshafen (DE)
• WISTUBA, Hermann
D-6800 Mannheim 51 (DE)
• OTTO, Bernhard
D-6703 Limburgerhof (DE)
• BUERGER, Gert
D-6800 Mannheim 24 (DE)
• HERRMANN, Guenter
D-6900 Heidelberg (DE)

(56) Entgegenhaltungen:
EP-A- 0 362 960     EP-A- 0 475 173
DE-A- 1 444 446     FR-A- 1 519 631
GB-A- 2 059 934

**Beschreibung**

Die Erfindung betrifft einen silberhaltigen Aluminiumoxid-Trägerkatalysator mit Kupferoxid und ein Verfahren zur katalytischen Zersetzung von reinem oder in Gasgemischen vorhandenem Distickstoffmonoxid bei erhöhter Temperatur.

Die katalytische Zersetzung von Distickstoffmonoxid (Lachgas) ist bereits lange bekannt und in zahlreichen Publikationen im Rahmen kinetischer Untersuchungen beschrieben. Bereits 1936 wird in Gmelins Handbuch der anorganischen Chemie, Stickstoff, 8. Auflage, Seite 573 ff, die Zersetzung von Distickstoffmonoxid im Quarzgefäß ohne und in Gegenwart von verschiedenen Katalysatoren wie $SiO_2$, Platinfolie, Titandioxid, Platinschwarz, $Al_2O_3$, Holzkohle sowie Thoriumdioxid bei verschiedenen Temperaturen untersucht. Kinetische Untersuchungen an reinem Silber, an Silber-Gold- und an Silber-Calcium-Legierungen werden von K.E. Hayes, Cand. J. Chem. 37, 583 (1959) beschrieben. R. Larsson gibt in Catalysis Today 4, 235-251 (1989) eine Übersicht über die Aktivierungsenergien der Distickstoffmonoxidzersetzung an oxidischen Katalysatoren, insbesondere an Mischoxiden.

Technisches Interesse an der Zersetzung von Distickstoffmonoxid zu Stickstoff und Sauerstoff ging von der NASA aus, die ausgehend von Ammonnitrat über Lachgas die Zersetzung in die Elemente nutzen wollte, um aus einer leicht handhabbaren Verbindung Atemluft für Astronauten zu erzeugen (Chem. Abstract 1481 (1965)). Als Katalysatoren wurden als geeignete beschrieben: Platin auf verschiedenen anorganischen Trägern, Rhodium auf Aluminiumoxid sowie Nickel-, Kobalt- und Molybdänoxide.

Ein weiteres technisches Anwendungsgebiet stellt die Beseitigung von Anasthesiegasen, z.B. in Operationsräumen dar. Zu diesem Applikationsfall gibt es eine Reihe japanischer Patentschriften. Folgende Katalysatorsysteme werden vorgeschlagen: Aktivkohle (in JP 54/11090), Elemente der Eisengruppe in Kombination mit Selten-Erd-Metallen (JP 61/45487), Platin, Palladium, Rhodium, Indium und/oder Ruthenium (JP 55/31463) und Kupfer-, Chromoxid auf Aluminiumoxid (JP 61/50650). Zersetzt wird in der Regel Distickstoffmonoxid in einem Distickstoffmonoxid-Luft-Gemisch (1:1).

Die JP 61/53142 beschreibt die Entfernung von Distickstoffmonoxid aus Abgasen an Kobalt-, Kupfer- und Manganoxiden auf $\gamma$-Aluminiumoxid.

In DE-OS 3 543 640 werden zur Zersetzung von Distickstoffmonoxid palladiumhaltige Katalysatoren vorgeschlagen.

Die JP 63/07826 beschreibt die Entfernung von Distickstoffmonoxid aus Abgasen an Edelmetallen bzw. Edelmetalloxiden auf $\gamma$-Aluminiumoxidträger. Die $N_2O$-Konzentration im Gas beträgt im Beispiel 26 ppm.

In den oben angeführten Publikationen werden durchweg Distickstoffmonoxid-Luft- bzw. Distickstoffmonoxid-Sauerstoff-Gemische zur Reaktion gebracht. Gemäß der DE-OS 3 543 640 werden auch Gemische mit 430 ppm $NO_x$ und 4 % Wasser (gasförmig) an Palladiumkatalysatoren umgesetzt.

FR-A-15 19 531 betrifft Katalysatoren, die elementares Kupfer bzw. Legierungen aus elementarem Kupfer mit Silber, Cobalt, Nickel, Palladium, Platin oder deren Gemischen auf $Al_2O_3$-Trägern und die 0,1 bis 1,5 % $Na_2O$ enthalten.

DE-A-14 44 446 betrifft Katalysatorsysteme aus Kupferoxid und Silberoxid aus Aluminiumoxidträgern.

In der DE-OS 40 29 061 werden silberhaltige Aluminumoxid-Trägerkatalysatoren zur Zersetzung von Distickstoffmonoxid beschrieben, wobei das Aluminiumoxid eine BET-Oberfläche von 5 bis 25 $m^2$/g aufweist.

In der nicht vorveröffentlichten Anmeldung DE 41 28 629 werden ebenfalls silberhaltige Aluminiumoxid-Trägerkatalysatoren zur Zersetzung von Distickstoffmonoxid beschrieben, die eine BET-Oberfläche von 26 bis 350 $m^2$/g aufweisen.

Aufgabe der vorliegenden Erfindung war es nun, ein Verfahren und einen Katalysator zur katalytischen Zersetzung von reinem oder in Gasgemischen enthaltenem Distickstoffmonoxid zur Verfügung zu stellen, mittels dessen Distickstoffmonoxid auch in Gegenwart höherer Konzentrationen von Stickoxiden und anderer Gase, die nicht Sauerstoff oder Stickstoff sind (insbesondere Abgase aus Adipinsäureanlagen), in die Elemente Stickstoff und Sauerstoff zersetzt werden kann, wobei der Katalysator eine höhere Aktivität als die bisher bekannten Katalysatoren aufweisen soll. Ein weiteres zu lösendes Problem ist dabei der selektive Abbau von Distickstoffmonoxid, ohne daß andere Stickoxide in nennenswertem Umfang in die Elemente zersetzt werden.

Es wurde nun gefunden, daß die Aufgabe mit Katalysatoren gelöst wird, die neben elementarem Silber und Aluminiumoxid Kupferoxid und gegebenenfalls einen oder mehrere Promotoren, ausgenommen Alkalimetalle, enthalten.

Gegenstand der vorliegenden Erfindung ist weiterhin ein Verfahren zur katalytischen Zersetzung von reinem oder in Gasgemischen vorhandenem Distickstoffmonoxid bei erhöhter Temperatur an silberhaltigen Aluminiumoxid-Trägerkatalysatoren, die neben elementarem Silber und Aluminiumoxid Kupferoxid und gegebenenfalls einen oder mehrere Promotoren, ausgenommen Alkalimetalle, enthalten.

Überraschenderweise setzt die $N_2O$-Zersetzungsreaktion an den erfindungsgemäßen Katalysatoren bereits bei einer deutlich niedrigeren Temperatur ein als bei den bekannten Katalysatoren.

Der Vorteil des erfindungsgemäßen Verfahrens besteht weiterhin darin, daß Distickstoffmonoxid sowohl in Reinstform als auch in Gemischen mit Sauerstoff oder Luft und in Gemischen mit Luft, die größere Mengen an Wasser und/oder größere Mengen an Stickoxiden (Stickstoffmonoxid, Stickstoffdioxid) enthalten, selektiv in die Elemente Stickstoff und Sauerstoff zersetzt werden kann, ohne daß dabei die höheren Stickstoffoxide abgebaut werden. Es gelingt problemlos, Distickstoffmonoxid in Gemischen mit z.B. bis zu 65 % Stickstoffdioxid ($NO_2$) und/oder 20 % Wasser in seine Elemente zu zersetzen.

Die erfindungsgemäßen Katalysatoren weisen im allgemeinen eine BET-Oberfläche von 0,1 bis 350 $m^2$/g auf.

Die Verfahren zur Herstellung des Trägers sind an sich Stand der Technik. Vorteilhaft erweist sich die Tränkung eines $Al_2O_3$-Trägers mit einer löslichen Kupferverbindung (z.B. Nitrat, Sulfat, Carboxylate u.a.) und anschließender thermischer Zersetzung des Anions zum Oxid. Eine weitere Möglichkeit wäre das Mischen einer Kupferverbindung mit einer Aluminiumverbindung (trocken oder in Suspension, insbesondere Sprühtrocknung), Verdichtung des Materials (z.B. durch Verkneten, gegebenenfalls Zugabe eines geeigneten Verformungshilfsmittels), Formgebung durch Extrudieren, Trocknung und anschließender Calcinierung zur Bildung der Oxide. Die Calciniertemperatur kann zwischen 300 und 1150°C liegen.

Zur Aufbringung des Silbers eignen sich im Prinzip alle Methoden, die in der Literatur zur Herstellung von Silberkatalysatoren vorgeschlagen wurden (z.B. D.I. Hucknall, Selective Oxidations of Hydrocarbons, Academic Press, London (1974), Seite 6). Vorteilhaft wendet man Methoden an, bei denen das Silber auf einen Träger aufgebracht wird. Beispielsweise kann eine Suspension von frisch gefälltem, gut gewaschenem Silberoxid (DE-AS 12 11 607) oder Silbercarbonat (US-PS 3 043 854) auf einen Träger aufgewälzt und die Silberverbindung anschließend thermisch zu Silber zersetzt werden. Bevorzugt eignet sich die Methode, einen grobporösen Träger mit der Lösung eines Silbersalzes (wie z.B. Silbernitrat (US-PS 3 575 888) oder Silberlactat (DE-AS 12 11 607) oder einer Silberkomplexverbindung (z.B. eines Silberamincarboxylat-Komplexes (DE-OS 21 59 346) zu tränken und anschließend durch Behandlung mit einem Reduktionsmittel oder durch Wärmebehandlung die Silberverbindung zu zersetzen.

Bevorzugte erfindungsgemäße Katalysatoren enthalten im allgemeinen 0,1 bis 40 Gew.-%, insbesondere 2 bis 25 Gew.-% Silber bezogen auf das Gewicht des Trägers. Größere Mengen Silber sind aus wirtschaftlichen Gründen uninteressant.

Die Silberpartikelgröße im ungebrauchten Katalysator liegt insbesondere zwischen 0,1 und 200 nm, bevorzugt zwischen 1 und 100 nm, besonders bevorzugt zwischen 2 und 50 nm. Die Bestimmung der Silberpartikelgröße kann z.B. durch XRD (X-ray diffraction) erfolgen.

Als Kupferoxid ist sowohl $Cu_2O$ als auch $CuO$ oder eine Mischung aus beiden Kupferoxiden einsetzbar.

Kupfer- und Aluminiumoxid können auch ganz oder teilweise in Form der Mischphase $CuAl_2O_4$ vorliegen.

Der Kupferoxidgehalt im Katalysator liegt bevorzugt im Bereich von 0,1 bis 90 Gew.-%, insbesondere bei 2 bis 60 Gew.-%, bezogen auf den Gesamtkatalysator.

Der Kupfer- und Aluminiumoxid-haltige Träger kann rein oder mit weiteren Elementen dotiert vorliegen. Die Dotierung von hochoberflächigen Trägern (insbesondere Aluminiumoxid-Träger) führt zur Erhöhung der thermischen Beständigkeit des Trägers (z.B. DE 3 403 328, DE 2 500 548, Appl. Catal 7, 211-220 (1983), J. Catal. 127, 595-604 (1991)). Zusätzlich sollen die Fremdionen zur katalytischen Aktivität des Katalysators beitragen. Zur Dotierung können die Verbindungen folgender Elemente herangezogen werden: Alkalimetalle, Erdalkalimetalle, Metalle der seltenen Erden, Metalle der 1. Übergangsmetallreihe, Y, Zr, B, Si und Zn. Der Gehalt des aluminiumoxidhaltigen Katalysators an Dotierverbindungen (Promotoren) kann zwischen 0,01 und 50 Gew.-% liegen.

Als Promotoren besonders geeignet sind Verbindungen der Elemente Mg, Ca, Sr, Ba, Co, Ni, Fe, Y und Ce. Besonders bevorzugt ist ZnO.

Die Porosität des Trägers sollte vorteilhaft so beschaffen sein, daß das Porenvolumen zwischen 0,01 und 0,8 ml/g liegt. Vorteilhaft liegen im Träger Meso- und/oder Makroporen vor. Unter Mesoporen werden dabei Poren mit einem Durchmesser von 2 bis 50 nm, unter Makroporen solche mit einem Durchmesser von über 50 nm verstanden.

Neben Silber können noch weitere metallische Aktivkomponenten, insbesondere Au, Pd, Pt, im Katalysator vorhanden sein.

Die erfindungsgemäßen Trägerkatalysatoren können in Form von Pellets, Waben, Ringen, Splitt, Voll- und Hohlsträngen oder auch in anderen geometrischen Formen ("shaped catalysts", siehe z.B. US 2 408 164, GB 2 193 907, US 4 233 187) vorliegen.

Für bestimmte Anwendungen ist es dabei wichtig, daß Form und Größe so gewählt werden, daß ein möglichst kleiner Druckverlust entsteht.

Bei dem erfindungsgemäßen Verfahren wird Distickstoffmonoxid oder ein Distickstoffmonoxid enthaltendes Gasgemisch bei erhöhten Temperaturen, bevorzugt von 200°C bis 900°C, über den Trägerkatalysator geleitet und dabei zu Stickstoff und Sauerstoff zersetzt. Dabei kann die GHSV (Gas Hour Space Velocity) zwischen 500 und 50.000 Nl Gas/l Kat. • h, vorzugsweise zwischen 1500 und 20.000 Nl Gas/l Kat. • h liegen.

Das erfindungsgemäße Verfahren kann so durchgeführt werden, daß man reines Distickstoffmonoxid oder Distickstoffmonoxid enthaltende Gasgemische oder Distickstoffmonoxid enthaltendes Abgas in einem Ofen oder Wärmetauscher auf die notwendige Reaktortemperatur vorheizt und dann durch ein mit dem erfindungsgemäßen Katalysator gefülltes Reaktionsrohr leitet. Das Vorheizen des Reaktionsgases kann auch direkt im Reaktionsrohr durch eine vorgeschaltete Inertmaterialschicht erfolgen. Zum Aufheizen des Katalysators und/oder des Inertmaterials kann neben einer externen Wärmequelle auch die Reaktionswärme, die bei der Zersetzung des Distickstoffmonoxids frei wird, herangezogen werden.

Im Gasgemisch, das nach dem erfindungsgemäßen Verfahren behandelt wird, können neben dem $N_2O$ insbesondere noch NO und/oder $NO_2$ vorhanden sein, und zwar insbesondere in einer Menge von 0,01 bis 50 Vol.-%, bezogen auf das Gesamtgas. Der $NO_2$-Gehalt des Gasgemisches beträgt bevorzugt 0,01 bis 50 Vol.-%, insbesondere 0,1 bis 30 Vol.-%. Das Gasgemisch kann neben $N_2O$ und $NO/NO_2$ ($NO_x$) außerdem noch $N_2$, $O_2$, CO, $CO_2$, $H_2O$ und Edelgase enthalten.

Der Druckbereich, in dem das Verfahren im allgemeinen durchgeführt wird, liegt zwischen 0,1 und 20 bar, d.h. es kann bei Unter- und Überdruck gearbeitet werden. Bevorzugt ist die Arbeitsweise bei Drücken von Normaldruck bis 7 bar.

Das Verfahren kann sowohl unter adiabatischen als auch unter isothermen Bedingungen durchgeführt werden.

Insbesondere eignet sich der erfindungsgemäße Katalysator und das erfindungsgemäße Verfahren dazu, um $N_2O$ in den Abgasen, die bei der Herstellung von Adipinsäure, z.B. durch Oxidation von Cyclohexanol und/oder Cyclohexanon mit Salpetersäure entstehen, zu zersetzen.

Ein besonderer Vorteil des Verfahrens liegt darin, daß $N_2O$ selektiv zersetzt wird, d.h. eine Zersetzung von $NO_x$ als Wertprodukt findet nicht statt.

Mit dem erfindungsgemäßen Katalysator und Verfahren gelingt es, die katalytische Zersetzung von Distickstoffmonoxid sowohl rein als auch in Gasgemischen bei deutlich tieferen Temperaturen durchzuführen, als es dem Stand der Technik entspricht. Die Erfindung stellt sowohl in wirtschaftlicher Hinsicht als auch in Bezug auf die Performance des Katalysators eine deutliche Verbesserung dar.

Beispiele

Typische Gaszusammensetzung:

| | |
|---|---|
| $N_2O$: | 23 Vol.-% |
| $NO_2$: | 17 Vol.-% |
| $N_2$: | 47 Vol.-% |
| $O_2$: | 7,5 Vol.-% |
| $H_2O$: | 3 Vol.-% |
| $CO_2$: | 2,5 Vol.-% |
| GHSV: | 4000 Nl Gas/l Kat. • h |

Die $N_2O$-Umsätze wurden nach folgender Formel berechnet:

$$\text{Umsatz in \%} = \frac{\text{Konz. am Gaseintritt - Konz. } N_2O \text{ am Gasaustritt}}{\text{Konz. } N_2O \text{ am Gaseintritt}} \times 100$$

Die Konz. $N_2O$ wurde jeweils gaschromatographisch bestimmt.

Herstellung der Katalysatoren

Beispiel 1

270 g AlO(OH) (Pural SB) wurden mit 30 g CuO und 10 g Ameisensäure (gelöst in 220 ml $H_2O$) 1 h verknetet, verstrangt, getrocknet und calciniert. 85,5 g hiervon (BET-Oberfläche 101 $m^2$/g; Wasseraufnahme 52,7 Gew.-%) wurden mit 45,19 ml wäßriger Lösung, die 23,8 g $AgNO_3$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator enthielt 14,4 Gew.-% Silber und hatte eine BET-Oberfläche von 94 $m^2$/g.

Beispiel 2

83,1 g handelsüblicher Aluminiumoxidträger (BET-Oberfläche 157 $m^2$/g; Wasseraufnahme 63,85 Gew.-%) wurde mit 53 ml wäßriger Lösung, die 28,1 g $Cu(NO_3)_2$ enthält, imprägniert und daraufhin eine Stunde bei Raumtemperatur stehengelassen. Der imprägnierte Träger wurde bis zur Gewichtskonstanz bei 120°C getrocknet und abschließend 4 h bei 600°C calciniert. Der so erhaltene Träger enthielt 10 Gew.-% CuO. 67,8 g des Kupferoxid-haltigen Aluminiumoxidträgers wurden mit 40,4 ml wäßriger Lösung, die 18,9 g $AgNO_3$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 600°C calciniert. Der so erhaltene Katalysator enthielt 14,9 Gew.-% Silber und hatte eine BET-Oberfläche von 104 $m^2$/g.

Beispiel 3

192,75 g handelsüblicher Aluminiumoxidträger (BET-Oberfläche 157 $m^2$/g; Wasseraufnahme 61,3 Gew.-%) wurde mit 122 ml wäßriger Lösung, die 65,05 g $Cu(NO_3)_2 \cdot 6\ H_2O$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 600°C calciniert.

73,58 g des mit Kupferoxid beladenen Aluminiumoxidträgers wurden mit 43 ml wäßriger Lösung, die 29,9 g $Zn(NO_3)_2 \cdot 6\ H_2O$ enthält, imprägniert, draufhin eine Stunde bei Raumtemperatur stehengelassen. Der imprägnierte Träger wurde bis zur Gewichtskonstanz bei 120°C getrocknet und abschließend 4 h bei 600°C calciniert. Der so erhaltene Katalysator enthielt 9,8 Gew.-% CuO und 9,9 Gew.-% ZnO. 78,7 g des Kupferoxid- und Zinkoxid-haltigen Aluminiumoxidträgers wurden mit 37 ml wäßriger Lösung, die 21,9 g $AgNO_3$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 600°C calciniert. Der so erhaltene Katalysator enthielt 14,8 Gew.-% Silber und hatte eine BET-Oberfläche von 75,8 $m^2$/g.

Beispiel 4

Eine Mischung aus 127,1 g AlO(OH) (Pural SB), 252,0 g thermisch vorbehandeltem $Al_2O_3$ (Puralox SCF A230) und 240,0 g CuO wurde unter Zugabe von 20 ml konzentrierter Ameisensäure in 210 ml $H_2O$ 1 h verknetet, verstrangt, getrocknet und calciniert. Der Träger enthält 39,8 Gew.-% CuO. 59,1 g hiervon wurden mit 29,5 ml wäßriger Lösung, die 16,4 g $AgNO_3$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator erhielt 14,6 Gew.-% Silber und hatte eine BET-Oberfläche von 63 $m^2$/g.

Beispiel 5

Eine Mischung aus 127,1 g AlO(OH) (Pural SB), 252,0 g $Al_2O_3$ wie in Beispiel 4 und 240,0 g CuO wurde unter Zugabe von 20 ml konzentrierter Ameisensäure in 210 ml $H_2O$ 1 h verknetet, verstrangt, getrocknet und calciniert. Der Träger enthält 39,8 Gew.-% CuO.

49,5 g hiervon wurden mit 32 ml wäßriger Lösung, die 78,8 g $Mg\ (NO_3)_2 \cdot 6\ H_2O$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert.

61,1 g hiervon wurden mit 27 ml wäßriger Lösung, die 17,0 g $AgNO_3$ enthält, imprägniert, daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator enthält 15,0 Gew.-% Silber und hatte eine BET-Oberfläche von 65 $m^2$/g.

Vergleichsbeispiel 1

Gemäß DE-OS 40 29 061 wurde ein Katalysator nachgestellt. 150 g handelsüblicher Aluminiumoxid-Träger (BET-Oberfläche 1,7 $m^2$/g; Wasseraufnahme 29,2 Gew.-%) wurde mit 100 ml wäßriger Lösung, die 41,7 g $AgNO_3$ enthält, imprägniert und daraufhin eine Stunde bei Raumtemperatur stehengelassen. Der imprägnierte Träger wurde bis zur Gewichtskonstanz bei 120°C getrocknet und abschließend 4 h bei 700°C calciniert. Der so erhaltene Katalysator enthielt 14,6 Gew.-% Silber und hatte eine BET-Oberfläche von 1,12 $m^2$/g.

Vergleichsbeispiel 2

Der gemäß DE-OS 35 43 640 bevorzugte Palladiumkatalysator auf alpha-Aluminiumoxid wurde nachgestellt. 200 g alpha-Aluminiumoxid (BET-Oberfläche 20,2 $m^2$/g) wurden mit NaOH imprägniert und bei 120°C getrocknet. Dieser Träger wurde mit 96 ml einer wäßrigen Natriumtetrachloropalladat-II-Lösung, enthaltend 1 g Pd, imprägniert und daraufhin 3 Stunden bei Raumtemperatur stehengelassen. Der $Pd^{2+}$-haltige Träger wurde zur Reduktion des $Pd^{2+}$ mit Hydrazin behandelt. Im Anschluß wurde der Katalysator chlorfrei gewaschen und bei 120°C bis zur Gewichtskonstanz getrocknet. Der so erhaltene Katalysator enthält 0,4 Gew.-% Palladium.

Vergleichsbeispiel 3

Gemäß DE 41 28 629 wurde ein Katalysator nachgestellt. 225 g AlO(OH) (Pural SB) wurden mit 25 g La(NO$_3$)$_3$ und 12,5 g Ameisensäure 3 h verknetet, verstrangt, getrocknet und calciniert. 64,10 g hiervon (BET-Oberfläche 183 m$^2$/g; Wasseraufnahme 76 Gew.-%) wurden mit 50,9 ml wäßriger Lösung, die 17,8 g AgNO$_3$ enthält, imprägniert und daraufhin eine Stunde bei Raumtemperatur stehen gelassen. Der imprägnierte Träger wurde bis zur Gewichtskonstanz bei 120°C getrocknet und anschließend 4 h bei 700°C calciniert. Der so erhaltene Katalysator enthielt 14,5 Gew.-% Silber und hatte eine BET-Oberfläche von 156 m$^2$/g.

Durchführung der Distickstoffmonoxid-Zersetzung

Als Versuchsapparatur diente ein 80 cm langes Reaktionsrohr aus Stahl der Güte Hasteloy C, unterteilt in Aufheiz- und Reaktionszone. Der Innendurchmessers beträgt 18 mm. Um den Temperaturverlauf im Rohr messen zu können, wurde ein Innenrohr mit 3,17 mm Außendurchmesser eingesetzt, indem ein Thermoelement leicht verschoben werden kann. Zur besseren Wärmeübertragung wurde der Reaktor in der Aufheizzone mit Inertmaterial (Steatit) gefüllt. Getestet wurden jeweils 40 ml Katalysator (Splitt 1,5-2 mm) bei Atmosphärendruck.

Versuchsergebnisse

| Katalysator | Laufzeit (h) | Temperatur (°C) | Umsatz (%) |
|---|---|---|---|
| 1 | 164 | 520 | > 99,9 |
| 2 | 242 | 490 | > 99,9 |
| 3 | 124 | 500 | > 99,9 |
| 4 | 150 | 500 | > 99,9 |
| 5 | 240 | 495 | > 99,9 |
| V1 | 150 | 610 | 97,5 |
| V2 | 112 | 640 | 66,5 |
| V3 | 280 | 530 | > 99,9 |

Die Ergebnisse zeigen, daß der erfindungsgemäße Katalysator zu hohen Umsätzen führt, obwohl die Umsetzungstemperatur niedriger liegt als bei den bekannten Katalysatoren. Die Versuche zeigen auch, daß die erfindungsgemäßen Katalysatoren eine gute Standzeit aufweisen.

**Patentansprüche**

1. Silberhaltiger Aluminiumoxid-Trägerkatalysator für die Zersetzung von reinem oder in Gasgemischen vorhandenem Distickstoffmonoxid, dadurch gekennzeichnet, daß der Trägerkatalysator neben elementarem Silber und Aluminiumoxid Kupferoxid und gegebenenfalls einen oder mehrere Promotor, ausgenommen Alkalimetalle, enthält.

2. Katalysator nach Anspruch 1, dadurch gekennzeichnet, daß er einen Kupferoxid-Gehalt von 0,1 bis 90 Gew.-% hat.

3. Katalysator nach den Anspruch 1, dadurch gekennzeichnet, daß der oder die Promotoren aus den Verbindungen folgender Elemente ausgewählt sind: Erdalkalimetalle, Metalle der seltenen Erden, Metalle der 1. Übergangsmetallreihe, Y, Zr, B, Si und Zn.

4. Katalysator nach Anspruch 1, dadurch gekennzeichnet, daß es sich bei den Promotoren um Verbindungen der Elemente Mg, Ca, Sr, Ba, Co, Ni, Fe, Y, Ce handelt.

5. Katalysator nach den Ansprüchen 1 bis 4, dadurch gekennzeichnet, daß er als zusätzlichen Promotor Zinkoxid enthält.

6. Katalysator nach den Ansprüchen 1 bis 5, dadurch gekennzeichnet, daß der Anteil der Promotoren 0,01 bis 50 Gew.-% beträgt.

7. Katalysator nach den Ansprüchen 1 bis 6, dadurch gekennzeichnet, daß die Porosität des Katalysators 0,01 bis 0,8 ml/g beträgt.

8. Katalysator nach den Ansprüchen 1 bis 7, dadurch gekennzeichnet, daß im Katalysator Mesoporen und/oder Makroporen vorliegen.

9. Katalysator nach den Ansprüchen 1 bis 8, dadurch gekennzeichnet, daß Kupferoxid und Aluminiumoxid ganz oder teilweise in Form der Mischphase $CuAl_2O_4$ vorliegen.

10. Katalysator nach den Ansprüchen 1 bis 9, dadurch gekennzeichnet, daß der Silbergehalt im Katalysator 0,1 bis 40 Gew.-% beträgt.

11. Katalysator nach den Ansprüchen 1 bis 10, dadurch gekennzeichnet, daß es sich bei den metallischen Aktivkomponenten um Au, Pd und/oder Pt handelt.

12. Verfahren zur katalytischen Zersetzung von reinem oder in Gasgemischen vorhandenem Distickstoffmonoxid bei erhöhter Temperatur an silberhaltigen Aluminiumoxid-Trägerkatalysatoren, dadurch gekennzeichnet, daß man einen Trägerkatalysator nach einem der Ansprüche 1 bis 11 einsetzt.

13. Verfahren nach Anspruch 12, dadurch gekennzeichnet, daß die Zersetzung in Gegenwart von 0,01 bis 65 Vol.-% Stickoxiden ($NO_x$) durchgeführt wird.

14. Verfahren nach den Ansprüchen 12 bis 13, dadurch gekennzeichnet, daß die Zersetzung bei Temperaturen von 200 bis 900°C durchgeführt wird.

15. Verfahren nach den Ansprüchen 12 bis 14, dadurch gekennzeichnet, daß der $N_2O$-Gehalt des Gasgemisches zwischen 0,01 und 65 Vol.-% liegt.

16. Verfahren nach den Ansprüchen 12 bis 15, dadurch gekennzeichnet, daß das Gasgemisch neben $N_2O$ und $NO_x$ noch $N_2$, $O_2$, CO, $CO_2$, $H_2O$ und/oder Edelgase enthält.

17. Verfahren nach den Ansprüchen 12 bis 16, dadurch gekennzeichnet, daß man die Zersetzung entweder unter adiabatischen oder isothermen Bedingungen durchführt.

18. Verfahren nach den Ansprüchen 12 bis 17, dadurch gekennzeichnet, daß es sich bei dem Gasgemisch um Abgas aus der Herstellung von Adipinsäure unter Mitverwendung von Salpetersäure handelt.

19. Verwendung eines Trägerkatalysators nach den Ansprüchen 1 bis 11 zur Zersetzung von Distickstoffmonoxid oder Distickstoffmonoxid in Gasgemischen.

## Claims

1. A silver-containing alumina supported catalyst for the decomposition of dinitrogen monoxide which is pure or present in gas mixtures, wherein the supported catalyst contains copper oxide, and one or more promoters where appropriate, excluding alkali metals, besides elemental silver and alumina.

2. A catalyst as claimed in claim 1, wherein the copper oxide content is from 0.1 to 90 % by weight.

3. A catalyst as claimed in claim 1, wherein the promoter or promoters are selected from the compounds of the following elements: alkaline earth metals, rare earth metals, metals of the first transition series, Y, Zr, B, Si and Zn.

4. A catalyst as claimed in claim 1, wherein the promoters are compounds of the elements Mg, Ca, Sr, Ba, Co, Ni, Fe, Y, Ce.

5. A catalyst as claimed in claims 1 to 4, which contains zinc oxide as additional promoter.

6. A catalyst as claimed in claims 1 to 5, which contains from 0.01 to 50 % by weight of promoters.

7. A catalyst as claimed in claims 1 to 6, wherein the porosity is from 0.01 to 0.8 ml/g.

8. A catalyst as claimed in claims 1 to 7, wherein mesopores and/or macropores are present.

9. A catalyst as claimed in claims 1 to 8, wherein copper oxide and alumina are present in whole or in part in the form of the mixed oxide $CuAl_2O_4$.

10. A catalyst as claimed in claims 1 to 9, wherein the silver content is from 0.1 to 40 % by weight.

11. A catalyst as claimed in claims 1 to 10, wherein the metal active components are Au, Pd and/or Pt.

12. A process for the catalytic decomposition of dinitrogen monoxide which is pure or present in gas mixtures at elevated temperature on silver-containing alumina supported catalysts, wherein a supported catalyst as claimed in any of claims 1 to 11 is employed.

13. A process as claimed in claim 12, wherein the decomposition is carried out in the presence of from 0.01 to 65 % by volume nitrogen oxides ($NO_x$).

14. A process as claimed in claim 12 or 13, wherein the decomposition is carried out at from 200 to 900°C.

15. A process as claimed in claims 12 to 14, wherein the $N_2O$ content in the gas mixture is from 0.01 to 65 % by volume.

16. A process as claimed in claims 12 to 15, wherein the gas mixture also contains $N_2$, $O_2$, CO, $CO_2$, $H_2O$ and/or inert gases besides $N_2O$ and $NO_x$.

17. A process as claimed in claims 12 to 16, wherein the decomposition is carried out either under adiabatic or isothermal conditions.

18. A process as claimed in claims 12 to 17, wherein the gas mixture is offgas from the preparation of adipic acid using nitric acid.

19. The use of a supported catalyst as claimed in claims 1 to 11 for decomposing dinitrogen monoxide or dinitrogen monoxide in gas mixtures.

**Revendications**

1. Catalyseur sur support à base d'oxyde d'aluminium contenant de l'argent pour la décomposition de protoxyde d'azote pur ou présent dans des mélanges de gaz, caractérisé en ce qu'il contient, en plus d'argent élémentaire et d'oxyde d'aluminium, de l'oxyde de cuivre et éventuellement un ou plusieurs promoteurs, à l'exception de métaux alcalins.

2. Catalyseur selon la revendication 1, caractérisé en ce qu'il a une teneur en oxyde de cuivre de 0,1 à 90% en poids.

3. Catalyseur selon la revendication 1, caractérisé en ce que le ou les promoteurs sont choisis parmi les composés des éléments suivants; métaux alcalino-terreux, métaux des terres rares, métaux de la 1ère série des métaux de transition, Y, Zr, B, Si et Zn.

4. Catalyseur selon la revendication 1, caractérisé en ce qu'il s'agit, en ce qui concerne les promoteurs, de composés des éléments Mg, Ca, Sr, Ba, Co, Ni, Fe, Y, Ce.

5. Catalyseur selon l'une quelconque des revendications 1 à 4, caractérisé en ce qu'il contient de l'oxyde de zinc en tant que promoteur supplémentaire.

6. Catalyseur selon l'une quelconque des revendications 1 à 5, caractérisé en ce que la proportion des promoteurs est comprise entre 0,01 et 50% en poids.

**7.** Catalyseur selon l'une quelconque des revendications 1 à 6, caractérisé en ce que la porosité du catalyseur est comprise entre 0,01 et 0,8 ml/g.

**8.** Catalyseur selon l'une quelconque des revendications 1 à 7, caractérisé en ce que des mésopores et/ou des macropores sont présents dans le catalyseur.

**9.** Catalyseur selon l'une quelconque des revendications 1 à 8, caractérisé en ce que l'oxyde de ouivre et l'oxyde d'aluminium sont présents en totalité ou en partie sous forme de la phase mixte $CuAl_2O_4$.

**10.** Catalyseur selon l'une quelconque des revendications 1 à 9, caractérisé en ce que la teneur du catalyseur en argent est comprise entre 0,1 et 40% en poids.

**11.** Catalyseur selon l'une quelconque des revendications 1 à 10, caractérisé en ce qu'il s'agit, en ce qui concerne les composants actifs métalliques, de Au, Pd et/ou Pt.

**12.** Procédé de décomposition catalytique de protoxyde d'azote pur ou présent dans des mélanges de gaz, à haute température et en présence de catalyseurs sur support à base d'oxyde d'aluminium contenant de l'argent, caractérisé en ce que l'on utilise un catalyseur sur support selon l'une quelconque des revendications 1 à 11.

**13.** Procédé selon la revendication 12, caractérisé en ce que la décomposition est effectuée en présence de 0,01 à 65% en volume d'oxydes de l'azote ($NO_x$).

**14.** Procédé selon la revendication 12 ou 13, caractérisé en ce que la décomposition est effectuée à des températures de 200 à 900°C.

**15.** Procédé selon l'une quelconque des revendications 12 à 14, caractérisé en ce que la teneur en $N_2O$ du mélange de gaz se situe entre 0,01 et 65% en volume.

**16.** Procédé selon l'une quelconque des revendications 12 à 15, caractérisé en ce que le mélange de gaz contient, en plus de $N_2O$ et de $NO_x$, $N_2$, $O_2$, CO, $CO_2$, $H_2O$ et/ou des gaz rares.

**17.** Procédé selon l'une quelconque des revendications 12 à 16, caractérisé en ce que l'on effectue la décomposition, soit dans des conditions adiabatiques, soit dans des conditions isothermes.

**18.** Procédé selon l'une quelconque des revendications 12 à 17, caractérisé en ce qu'il s'agit, en ce qui concerne le mélange de gaz, de gaz résiduaire provenant de la préparation d'acide adipique avec utilisation simultanée d'acide nitrique.

**19.** Utilisation d'un catalyseur sur support selon l'une quelconque des revendications 1 à 11 pour la décomposition de protoxyde d'azote ou de protoxyde d'azote dans des mélanges de gaz.